# EUROPÄISCHE PATENTSCHRIFT

(11) **EP 2 776 017 B1**
(45) Veröffentlichungstag und Bekanntmachung des Hinweises auf die Patenterteilung: **20.12.2017**
(21) Anmeldenummer: 12781309.5
(22) Anmeldetag: 01.11.2012
(51) Int. Cl.: A61K 31/00, A61P 35/00, C07F 1/12

(54) **GOLDVERBINDUNGEN MIT ALKINYL-LIGANDEN UND DEREN THERAPEUTISCHE VERWENDUNG**
GOLD COMPOUNDS HAVING ALKYNYL LIGANDS AND THERAPEUTIC USE THEREOF
COMPOSÉS D'OR CONTENANT DES LIGANDS ALCYNYLIQUES ET LEUR UTILISATION THÉRAPEUTIQUE

(30) Priorität: 01.11.2011 DE 102011054972
(43) Veröffentlichungstag der Anmeldung: 17.09.2014
(73) Patentinhaber: Technische Universität Braunschweig Carolo-Wilhelmina, 38106 Braunschweig (DE); Ruprecht-Karls-Universität Heidelberg, 69117 Heidelberg (DE)
(72) Erfinder: BAGOWSKI, Christoph, 80804 München (DE); OTT, Ingo, 38114 Braunschweig (DE); MEYER, Andreas, 38104 Braunschweig (DE); WÖLFL, Stefan, 69120 Heidelberg (DE); KITANOVIC, Igor, 69120 Heidelberg (DE); CAN, Suzan, 69120 Heidelberg (DE); ALBORZINIA, Hamed, 69120 Heidelberg (DE)
(74) Vertreter: Kröncke, Rolf
(86) Internationale Anmeldenummer: PCT/EP2012/071657
(87) Internationale Veröffentlichungsnummer: WO 2013/064594

(56) Entgegenhaltungen:
- US-A1- 2006 269 779
- US-A1- 2007 142 336
- JOÃO CARLOS LIMA ET AL: "Applications of gold(i) alkynyl systems: a growing field to explore", CHEMICAL SOCIETY REVIEWS, Bd. 40, Nr. 11, 18. Juli 2011 (2011-07-18) , Seite 5442, XP055047245, ISSN: 0306-0012, DOI: 10.1039/c1cs15123a
- ROBERT A. STOCKLAND ET AL: "Organometallic Complexes Containing 17-Ethynyl-17[beta]-hydroxyandrost-4-en-3- one and Related Ethynyl Steroids", ORGANOMETALLICS, Bd. 25, Nr. 10, 1. Mai 2006 (2006-05-01), Seiten 2475-2485, XP055047285, ISSN: 0276-7333, DOI: 10.1021/om051064r
- SCHUH ET AL.: "Synthesis and biological studies of some gold(I) complexes containing functionalised alkynes", DALTON TRANS., no. 48, 8 October 2009 (2009-10-08), pages 10841-10845, XP055263442, GB ISSN: 1477-9226, DOI: 10.1039/b913896j
- CHUI ET AL.: "Antitumor activity of diethynylfluorene derivatives of gold(I)", BIOORG. MED. CHEM., vol. 17, no. 23, 1 December 2009 (2009-12-01), pages 7872-7877, XP026742055, ISSN: 0968-0896, DOI: 10.1016/J.BMC.2009.10.034

## Beschreibung

Die vorliegende Erfindung betrifft in einem ersten Aspekt neue Angiogenesehemmer zur Behandlung von Krebs auf Basis von Gold(I)-Alkinkomplexen. Genauer betrifft die vorliegende Anmeldung die Verwendung von Verbindungen mit einer Phosphan- oder Carbengruppe, Au(I), einer Alkinyl-Einheit und einem, gegebenenfalls über einen Linker mit der Alkinyl-Einheit verbundenen Aromaten. Diese Verbindungen zeigen eine hervorragende Hemmung der Angiogenese in einem Individuum, starke antiproliferative Wirksamkeit, Effekte auf den Sauerstoffverbrauch von Mitochondrien, sowie eine Hemmwirkung des Enzyms Thioredoxin-Reduktase, wodurch der Einsatz in der Tumortherapie möglich ist. Weiterhin betrifft die vorliegende Anmeldung neue pharmazeutische Zusammensetzungen enthaltend Carben-Gold(I)-Alkin-Komplexe.

### Stand der Technik

Gold(I)-Verbindungen werden seit mehreren Jahrzehnten zur Therapie der rheumatoiden Arthritis eingesetzt. Weitere derzeit intensiv beforschte Anwendungsmöglichkeiten finden sich in der Behandlung von Tumoren sowie von Infektionskrankheiten und hier insbesondere parasitäre und bakterielle Erkrankungen.

Die zur Therapie zugelassenen Gold(I)-Verbindungen, wie etwa Natriumaurothiomalat oder Auranofin, stellen jedoch relativ instabile Wirkstoffe dar, die zu aktiven Metaboliten umgewandelt werden.

Der starke Metabolismus hat bisher auch die gezieltere Weiterentwicklung dieser Verbindungsklasse weitgehend verhindert und führt generell zu einem sehr komplexen pharmakodynamischen Verhalten mit therapierelevanten Nebenwirkungen (z.B. Hautreaktionen, Blutbildveränderungen). Der derzeitige therapeutische Einsatz dieser Verbindungen beschränkt sich daher weitgehend auf die Behandlung der rheumatoiden Artritis (sog. "Basistherapie" mit *disease modifying antirheumatic drugs,* DMARDs). Die Entwicklung neuer Goldverbindungen mit verbesserten pharmakologischen Eigenschaften ist daher von großem Interesse.

Ein Überblick hierzu findet sich zum Beispiel in dem Übersichtsartikel von Ott I., Coordination Chemistry Reviews, 2009, 253, 1670-1681. In diesem Übersichtsartikel werden verschiedenste Gold(I)-, Gold(II)-, Gold(III)-Komplexe und ihre möglichen pharmazeutischen Anwendungen diskutiert. Organometallische Antikrebsverbindungen werden weiterhin im Übersichtsartikel von Gasser G. et.al., J. Med. Chem., 2011, 54, 3-25 beschrieben.

Chui C.-H., et.al., Bioorganic & Medicinial Chemistry, 2009, 17, 7872-7877 beschreibt eine Antitumoraktivität von Diethinylfluoren-Derivaten von Gold(I). Die dort beschriebenen zwei Goldverbindungen leiten sich von Diethinylfluoren ab und tragen jeweils zwei Gold(I)-triethylphosphan-Gruppen. Die Antitumoraktivität der Verbindungen ist in Tumorzelllinien und Mäusen beschrieben.

Schuh E., et. al., Dalton Trans., 2009, 10841-10845 beschreiben die Synthese und biologische Studien zu Gold(I)-Komplexen enthaltend funktionalisierte Alkine. Diese funktionalisierten Alkine zeigen Anti-Malaria-Aktivität sowie eine gute Zytotoxizität in Tumorzellen. Vergara et. al., Organometallics, 2010, 29, 2596-2603 beschreiben anti-proliferative Wirkungen von Gold(I)-Alkin-Komplexen mit wasserlöslichen Phosphanresten. Die dort beschriebenen Verbindungen zeigen eine gute Zytotoxizität in Tumorzellen.

US 2006/269779 A1 beschreibt organische Polymere aufweisende lichtemittierende Materialien mit Gold-Komplexen.

Lima J.C. et al, Chem. Soc. Rev. 2011, 40, 5442-5456 beschreibt Anwendungen von Gold(I), Alkinyl-Systemen.

Stockland et. al., Organometallics, 2006, 25, 2475-2485, beschreibt Organometallkomplexe enthaltend steroidhaltige Verbindungen.

Es besteht nach wie vor der Bedarf an Gold(I)-Komplexen, insbesondere an stabilen Gold(I)-Komplexen, die unter physiologischen Bedingungen sowohl eine ausreichende Stabilität aber auch Bioverfügbarkeit aufweisen, um ihre pharmazeutisch wirksame Aktivität zu entfalten.

Erfindungsgemäß werden Gold(I)-Komplexe bereitgestellt, die eine ausgezeichnete Angiogenese-Hemmung aufweisen, geeignet zur Behandlung von Krebs. Die Verbindungen zeichnen sich dadurch aus, dass sie zudem starke Hemmstoffe des Enzyms Thioredoxin-Reduktase sind. Diese Verbindungen sind als Angiogenesehemmer in der Tumortherapie geeignet.

### Kurze Beschreibung der Erfindung

In einem ersten Aspekt wird die Verwendung von Verbindungen der allgemeinen Formel (I): 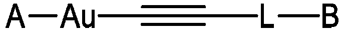
wobei A ein Substituent ist ausgewählt aus der Gruppe (II), (III) oder (IV): 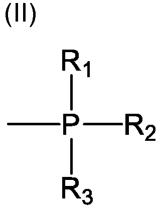 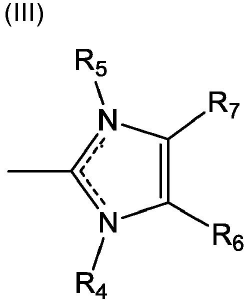 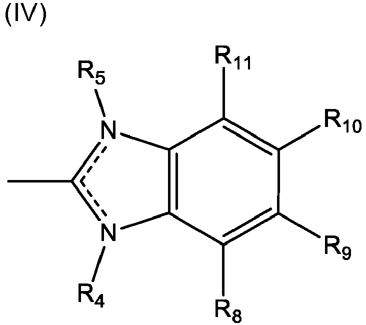
wobei R₁, R₂ und R₃ unabhängig voneinander ausgewählt ist aus CH₂-CH₃, einem gegebenenfalls substituiertem Phenylrest oder einem gegebenenfalls substituiertem Furanylrest, wobei dieser substituiert sein kann mit Substituenten ausgewählt aus Halogen, C₁-C₃ Alkyl, Hydroxyl, OC₁-C₃ Alkyl, -NH₂, -N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂,
wobei R₄, R₅, R₆, R₇, R₈, R₉, R₁₀, R₁₁, unabhängig voneinander ausgewählt ist aus der Gruppe bestehend aus Wasserstoff, Hydroxyl, linearen oder verzweigten C₁-C₆ Alkyl, Hydroxy-C₁-C₆ Alkyl, C₁-C₆ Alkoxy, C₁-C₆ Alkyl-C₁-C₆ Alkoxy, Benzyl, oder gegebenenfalls substituierten aromatischen Rest, wobei dieser aromatische Rest substituiert sein kann mit Substituenten ausgewählt aus Halogen, C₁-C₃ Alkyl, Hydroxyl, OC₁-C₃ Alkyl, -NH₂, -N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂,
L stellt eine Linkergruppe dar, die fehlt.
B ist ein gegebenenfalls substituierter aromatischer Rest, wobei dieser aromatische Rest substituiert sein kann mit Substituenten ausgewählt aus C₁-C₃ Alkyl, Hydroxyl, OC₁-C₃ Alkyl, -NH₂, -N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂, Halogen, und deren pharmazeutisch annehmbaren Salze zur Verwendung als Angiogenesehemmer zur Behandlung von Krebs.

Weiterhin wird die Verwendung einer Verbindung beschrieben der allgemeinen Formel (I): 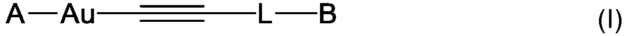
wobei A eine Gruppe (II)ist, wobei R₁, R₂, und R₃ ein gegebenenfalls substituierter Furanylrest ist, wobei die Substituenten aus den unten genannten Substituenten ausgewählt sein können, oder A ist eine Gruppe (III) oder (IV),
wobei R₄, R₅, R₆, R₇, R₈, R₉, R₁₀, R₁₁ unabhängig voneinander ausgewählt sind aus der Gruppe bestehend aus Wasserstoff, Hydroxyl, linearen oder verzweigten C₁-C₆ Alkyl, Hydroxy-C₁-C₆ Alkyl, C₁-C₆ Alkoxy, C₁-C₆ Alkyl-C₁-C₆ Alkoxy, C₁-C₆ Aralkyl, insbesondere Benzyl oder Phenyl, oder gegebenenfalls substituierten aromatischen Rest, der gegebenenfalls Heteroatome aufweisen kann, wobei dieser aromatische Rest substituiert sein kann mit Substituenten ausgewählt aus Halogen (F, Cl, Br, I), C₁-C₃ Alkyl, OC₁-C₃ Alkyl, Hydroxyl, -NH₂,-N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂,
L stellt eine Linkergruppe dar, wie oben definiert;
B ist ein gegebenenfalls substituierter aromatischer Rest, der gegebenenfalls Heteroatome aufweisen kann, wobei dieser aromatische Rest substituiert sein kann mit Substituenten ausgewählt aus C₁-C₃ Alkyl, Hydroxyl, OC₁-C₃ Alkyl,-NH₂, -N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂, Halogen (F, Cl, Br, I), oder pharmazeutisch annehmbare Salze hiervon als zytotoxische Verbindung, insbesondere zur Behandlung von Krebs.

Weiterhin werden erfindungsgemäß pharmazeutische Zusammensetzungen enthaltend Verbindungen der allgemeinen Formel (I), wobei A ein Substituent der allgemeinen Formel (III), oder (IV) ist, bereitgestellt. Es handelt sich bei diesen Verbindungen um Angiogenesehemmer zur Behandlung von Krebs.

### Kurze Beschreibung der Abbildungen

Figur 1: In der Figur 1 sind die in den Beispielen 1 bis 6 synthetisierten Verbindungen dargestellt.
Figur 2. Messung der Sauerstoffsättigung über die Zeit an isolierten Mitochondrien (Mausleber) nach Behandlung mit dem Gold(I)-Komplex gemäß Beispiel 1 in zwei Konzentrationen und DMF als Kontrolle.

### Ausführliche Beschreibung der Erfindung

In einem ersten Aspekt werden Verbindungen geeignet zur Hemmung der Angiogenese in einem Individuum bereitgestellt. Bei diesen Verbindungen handelt es sich um solche der allgemeinen Formel (I): 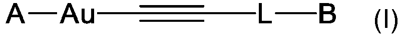
wobei A ein Substituent ist ausgewählt aus der Gruppe (II), (III) oder (IV): 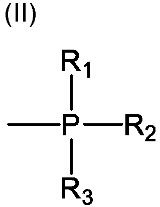 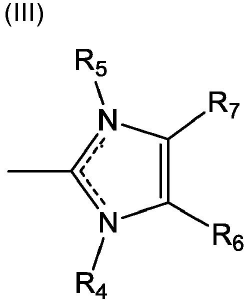 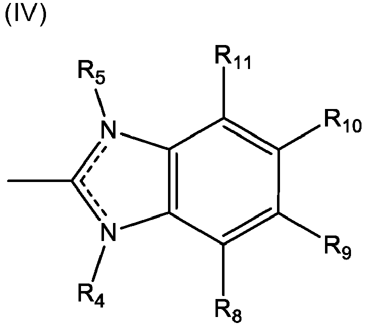
wobei R₁, R₂ und R₃ unabhängig voneinander ausgewählt ist aus CH₂-CH₃, einem gegebenenfalls substituiertem Phenylrest oder einem gegebenenfalls substituiertem Furanylrest, wobei dieser substituiert sein kann mit Substituenten ausgewählt aus Halogen, C₁-C₃ Alkyl, Hydroxyl, OC₁-C₃ Alkyl, -NH₂, -N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂,
wobei R₄, R₅, R₆, R₇, R₈, R₉, R₁₀, R₁₁, unabhängig voneinander ausgewählt ist aus der Gruppe bestehend aus Wasserstoff, Hydroxyl, linearen oder verzweigten C₁-C₆ Alkyl, Hydroxy-C₁-C₆ Alkyl, C₁-C₆ Alkoxy, C₁-C₆ Alkyl-C₁-C₆ Alkoxy, Benzyl, oder gegebenenfalls substituierten aromatischen Rest, wobei dieser aromatische Rest substituiert sein kann mit Substituenten ausgewählt aus Halogen, C₁-C₃ Alkyl, Hydroxyl, OC₁-C₃ Alkyl, -NH₂, -N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂,
L stellt eine Linkergruppe dar, die fehlt.
B ist ein gegebenenfalls substituierter aromatischer Rest, wobei dieser aromatische Rest substituiert sein kann mit Substituenten ausgewählt aus C₁-C₃ Alkyl, Hydroxyl, OC₁-C₃ Alkyl, -NH₂, -N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂, Halogen, und deren pharmazeutisch annehmbaren Salze zur Verwendung als Angiogenesehemmer zur Behandlung von Krebs.

In einem weiteren Aspekt richtet sich die vorliegende Erfindung auf die Verwendung einer Untergruppe der Verbindungen der allgemeinen Formel (I), bei denen der Substituent A einer der allgemeinen Formel (III) oder (IV) ist, oder bei denen der Substituent eine Gruppe (II) wobei R₁, R₂ und R₃ ein gegebenenfalls substituierter Furanylrest ist, ist. Diese Verbindungen eignen sich in der Verwendung als Verbindungen zur Behandlung von Krebs.

Vorliegend wurde gefunden, dass die Verbindungen der allgemeinen Formel (I) umfassend einen Phosphan-Rest gemäß Formel (II) z.B. mit Furanylresten oder Phenylresten als R₁, R₂ und/oder R₃, oder einen N-heterozyklischen CarbenRest gemäß Formel (III) oder (IV),einem Au(I), sowie als weiteres Element der erfindungsgemäßen Verbindungen eine Alkinyl-Einheit, die mit dem Au(I) verbunden ist, eine anti-angiogene Eigenschaft aufzeigen. Erfindungsgemäß ist dabei die Reihenfolge der einzelnen Elemente Phosphan-/ Carbenrest-Au(I)-Alkin-. Das Alkin ist weiterhin mit einem aromatischen Rest, der gegebenenfalls substituiert sein kann, verbunden.

Die erfindungsgemäßen Verbindungen zeigen überraschend eine Angiogenese hemmende Wirkung auf. Die Verbindungen sind daher erfindungsgemäß insbesondere dazu geeignet die Angiogenese in einem Individuum zur Behandlung von Krebs zu hemmen. Weiterhin zeigte insbesondere eine Untergruppe der Verbindung der allgemeinen Formel (I), wobei der Substituent A als ein Carbenrest gemäß Formel (III) oder (IV), oder ein Phosphanrest gemäß Gruppe (II) mit einem Furanylrest als R₁, R₂ und R₃ vorliegt, hervorragende zytotoxische Eigenschaften und sind somit entsprechend geeignet als Antitumorwirkstoffe eingesetzt zu werden.

Die Erfinder konnten dabei zeigen, dass die erfindungsgemäßen Koordinationsverbindungen mit einem Gold(I)-Zentralatom sowie einer Alkinyl-Einheit, das mit einem gegebenenfalls substituierten Aromaten verbunden ist und einem Phosphan als Liganden eine gute Stabilität auch unter physiologischen Bedingungen aufweisen. Des Weiteren konnte gezeigt werden, dass die erfindungsgemäßen Verbindungen hochselektiv das Enzym Thioredoxin-Reduktase hemmen. Überraschend zeigte sich dabei die antiangiogene Wirkung. Dadurch ist es möglich gezielt Angiogenese in einem Individuum zu hemmen und somit die Entwicklung und von z.B. neoplastischen Gewebe zu hemmen, um Krebs zu behandeln.

L ist nicht vorhanden.

Weiterhin ist es bevorzugt, dass der Substituent B, der Aromat, ausgewählt ist aus 2,3-Dihydro-1,3-dioxo-1H-benzo[de]isochinolin, 2,3-Dihydro-1H-benzo[de]isochinolin, 1,3-Dioxoisoindolin, Benzol, sowie Anthracen, Phenanthren, Pyridin, Chinolin, Isochinolin, Indol, Imidazol, Furan, Thiophen, Benzofuran oder Benzothiophen, die gegebenenfalls substituiert sein können.

Insbesondere ist es bevorzugt, dass, wenn A ein Substituent der allgemeinen Formel (III) oder (IV) ist (d.h. ein Carbenrest), R₄, R₅, R₆, R₇, R₈, R₉, R₁₀, R₁₁ unabhängig voneinander ausgewählt sind aus Methyl, Ethyl, Propyl oder Butyl oder nicht vorhanden sind, d.h. ein Wasserstoff darstellt.

Wenn der Substituent A einer der allgemeinen Formel (II) ist, ein Phosphanrest, ist dabei R₁, R₂, und R₃ bevorzugt unabhängig voneinander ausgewählt aus CH₂-CH₃, Phenyl und Furanyl, das gegebenenfalls substituiert sein kann. Insbesondere ist es bevorzugt, dass ein gegebenenfalls substituierter Phenylrest ist.

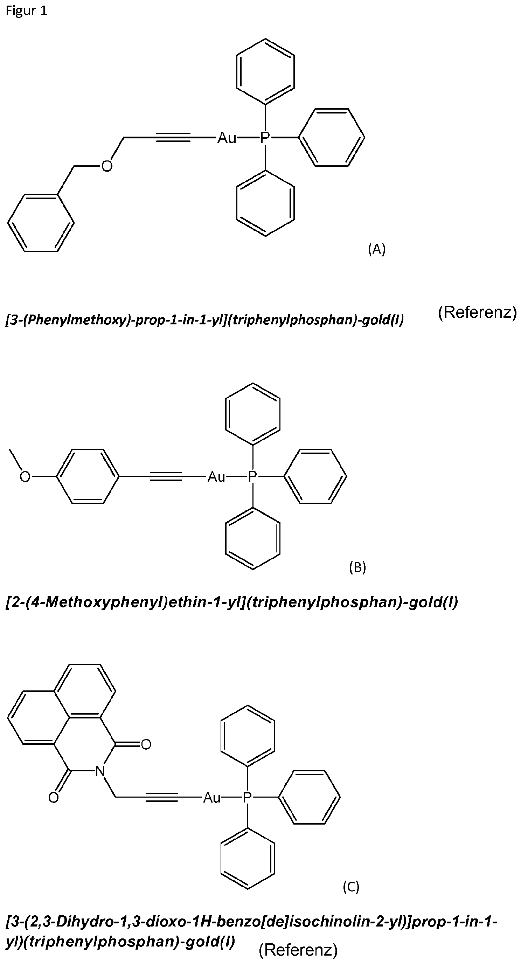

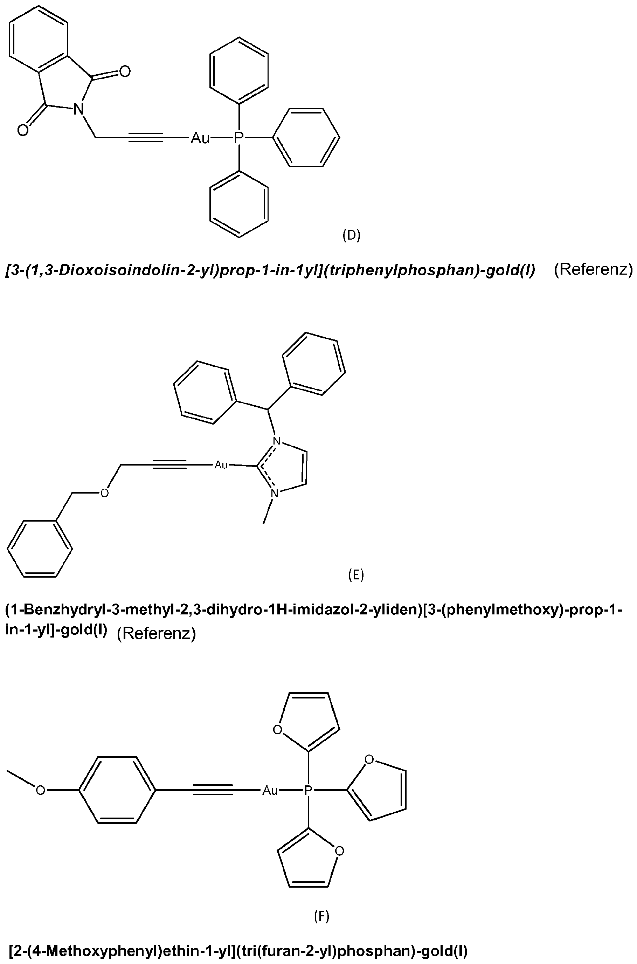

Geeignete Verbindungen sind z.B. die Verbindungen (B) und (F), gezeigt in der Figur 1.

D.h., besonders geeignet zur erfindungsgemäßen Verwendung sind Verbindungen, wobei A ein Substituent (II) ist mit R₁, R₂ und R₃ ist ein Phenylrest oder R₁, R₂ und R₃ ist ein Furanylrest. Weiterhin ist es bevorzugt, dass wenn A ein Substituent der Gruppe (III) ist, R₆ und R₇ H sind und mindestens eines aus R₄ und R₅ einen Aromaten aufweist, wie einen Phenylrest, einen Benzylrest oder einen Benzhydrylrest.

In einem weiteren Aspekt richtet sich die vorliegende Anmeldung auf die Verwendung der beschriebenen Verbindungen als Angiogenesehemmer zur Behandlung von Krebs, wobei die Verbindungen insbesondere zur Verabreichung über den oralen oder intravenösen Weg angepasst sind.

In einem weiteren Aspekt richtet sich die vorliegende Anmeldung auf pharmazeutische Zusammensetzungen enthaltend eine Verbindung der allgemeinen Formel (I), wobei A ein Substituent der allgemeinen Formel (III) oder (IV) ist oder A ist ein Substituent der Gruppe (II) wobei R₁, R₂ und R₃ einen gegebenenfalls substituierten Furanylrest darstellen, wie vorliegend definiert. Die pharmazeutischen Eigenschaften solcher Verbindungen sind bisher noch nicht beschrieben. Vorliegend konnte das erste Mal gezeigt werden, dass diese Verbindungen der allgemeinen Formel (I), wobei A ein Substituent der allgemeinen Formel (III) oder (IV) ist, oder A ist ein Substituent der Gruppe (II) wobei R₁, R₂ und R₃ einen gegebenenfalls substituierten Furanylrest darstellen, pharmazeutische, nämlich angiogenesehemmende aber auch zytotoxische Eigenschaften aufweisen.

Vorliegend konnte gezeigt werden, dass die Verbindungen, wie sie hierin beschrieben werden, antiproliferative Aktivitäten und Effekte auf die mitochondriale Atmung aufweisen. Weiterhin konnten anti-angiogene Eigenschaften, d.h. eine Hemmung der Blutgefäßneubildung, gezeigt werden.

Insbesondere sind die erfindungsgemäß beschriebenen Komplexe wirksame und selektive Hemmer der Thioredoxin-Reduktase und zeigen hervorragende anti-angiogene Wirkungen.

Weiterhin wurde festgestellt, dass die erfindungsgemäßen Verbindungen eine gute Stabilität aufzeigen und dabei selektiv TrxR hemmen.

Die Erfinder konnten schließlich zeigen, dass entsprechende Verbindungen nicht nur auf die Zellen selbst, einschließlich neoplastischen Zellen, zytotoxische Wirkungen haben. Überraschenderweise zeigten die erfindungsgemäßen Verbindungen weiterhin anti-angiogene Eigenschaften, d.h. die Ausbildung von Blutgefäßen wird verhindert, so dass entsprechende Gewebe, z.B. solche mit neoplastischen Zellen, nicht weiter mit Nährstoffen versorgt werden. Insbesondere ein Vergleich mit metallfreien Liganden, die als Referenz ebenfalls geprüft wurden, zeigte die Relevanz der Goldkomponente bei Aufzeigen der anti-angiogenen Wirkung. Selbst im Vergleich zu bekannten anti-angiogenen Stoffen, wie Thalidomid, zeigten die erfindungsgemäßen Verbindungen überragende Eigenschaften. Die erfindungsgemäßen Verbindungen sind daher zur Verwendung als anti-angiogene Arzneimittel geeignet.

Die pharmazeutischen Zusammensetzungen gemäß der vorliegenden Erfindung können übliche, pharmazeutisch annehmbare Hilfsstoffe enthalten, wie Verdünner, Exzipienten oder Träger.

Die erfindungsgemäßen Verbindungen können dabei je nach Verabreichungsweg bevorzugt in dem Fachmann bekannten, üblichen Mengen eingesetzt werden. Dem Fachmann sind entsprechende Mittel und Verfahren bekannt, die geeigneten Dosen zu ermitteln. Die Verbindungen können zur Anwendung entsprechend bekannter Verfahren formuliert sein. Insbesondere können die pharmazeutischen Zusammensetzungen weitere Hilfsmittel, wie Träger, Verdünner, Exzipienten und andere Hilfsstoffe enthalten.

Die Verwendung der erfindungsgemäßen Verbindungen kann dabei alleine oder in Kombination mit bekannten Angiogenesehemmern oder anderen Zytostatika oder Krebsmitteln durchgeführt werden. Hemmstoffe der Angiogenese dienen dazu die Neubildung von Gefäßen zu unterdrücken. Dadurch wird das Wachsen von Tumoren gebremst. Die Wirkung von Zytostatika ist direkt gegen das Wachsen von Tumorzellgewebe gerichtet.

### Beispiele

### Synthesechemie

Zu einer Lösung des entsprechenden Alkins (0,1-0,2 mmol in 8,0 ml Methanol/ Ethanol 1:1) wurde ein Volumen von 0,2 ml einer 2,0 M methanolischen KOH-Lösung hinzugefügt und die erhaltene Lösung wurde für 5 Minuten bei Raumtemperatur gerührt. Chlor(triphenylphosphin)gold(I) (ClAuPPh₃) (0,1-0,2 mmol, 49,5 mg) wurden hinzugefügt und das Rühren wurde für 3 Tage fortgesetzt. Das blassgelbe Präzipitat wurde mittels Filtration isoliert und mit 2,0 ml MeOH gewaschen.

### Beispiel 1: [3-(Phenylmethoxy)-prop-1-in-1-yl](triphenylphosphan)-gold(I)(A) (Referenz)

Ausbeute: 25,4 mg (0,042 mmol, 42%) blassgelbe Kristalle
¹H NMR (400 MHz, CDCl₃) δ 4,37 (s, 2H, C*H*₂-O-CH₂-Ph), δ 4,70 (s, 2H, C*H*₂-Ph), δ 7,14-7,79 (m, 20H, Ar*H*). ³¹P NMR (162 MHz, CDCl₃): δ 42,7. IR (KBr Pressling) 2118 cm⁻¹ (C=C). Elementaranalyse für C₂₈H₂₄AuOP (% Soll/Ist): C (55,64/55,65), H (4,00/3,92), N (0,00/0,00).

### Beispiel 2: [2-(4-Methoxyphenyl)ethin-1-yl](triphenylphosphan)-gold(I)(B)

Ausbeute: 31,3 mg (0,053 mmol, 53%) blassgelbe Kristalle
¹HNMR (400 MHz, CDCl₃) δ 3,79 (s, 3H, C*H*₃), δ 6,79 (d, J=9,0 Hz, 2H, Ar*H*₂-₃), δ 7,33-7,89 (m, 17H, Ar*H*). ³¹P NMR (162 MHz, CDCl₃): δ 43,0. IR (KBr Pressling) 2109 cm⁻¹ (C≡C). Elementaranalyse für C₂₇H₂₂AuOP (% Soll/Ist): C (54,93/54,88), H (3,76/3,71), N (0,00/0,00)

### Beispiel 3:

### [3-(2,3-Dihydro-1,3-dioxo-1H-benzo[de]isochinolin-2-yl)]prop-1-in-1-yl)(triphenylphosphan)-gold(I) (C) (Referenz)

Ausbeute: 80,0 mg (0,120 mmol, 57%) blassgelbe Kristalle
¹HNMR (CDCl₃, 400 MHz) δ 5,12 (s, 2H, C*H*₂), δ 7,26-8,63 (m, 21H, Ar*H*). ³¹P NMR (162 MHz, CDCl₃): δ 42,6. Elementaranalyse für C₂₇H₂₂AuOP (% Soll/Ist): C (57,15/56,58), H (3,34/3,27), N (2,02/1,82).

### Beispiel 4

### [3-(1,3-Dioxoisoindolin-2-yl)prop-1-in-1yl](triphenylphosphan)-gold(I) (D) (Referenz)

Ausbeute: 41,0 mg (0,064 mmol, 32%) blassgelbe Kristalle
¹H NMR (CDCl₃, 400 MHz) δ 4,61 (s, 2H, C*H*₂), δ 7,35-7,90 (m, 19H, Ar*H*). IR (KBr Pressling) 2133 cm⁻¹ (C≡C). ³¹P NMR (162 MHz, CDCl₃): δ 42,6. Elementaranalyse für C₂₇H₂₂AuOP(% Soll/Ist): C (54,13/54,85), H (3,29/3,09), N (2,18/1,65).

### Beispiel 5

### (1-Benzhydryl-3-methyl-2,3-dihydro-1H-imidazol-2-yliden)[3-(phenylmethoxy)-prop-1-in-1-yl]-gold(I) (E) (Referenz)

Ausbeute: 36,0 mg (0,061 mmol, 47%) gelbliche Kristalle
¹H NMR (CD₂Cl₂, 400 MHz) δ 3,86 (s, 3H, C*H*₃), δ 4,25 (s, 2H, C*H*₂-Ph), δ 4,58 (s, 2H, C*H*₂-O-CH₂-Ph), δ 6,81 (d, J=1,9 Hz, 1H, C*H*-NCH₃), δ 6,93 (d, J=1,9 Hz, 1H, C*H*-NCHPh₂), δ 7,35-7,90 (m, 15H, Ar*H*), δ 7,35-7,90 (s, 1H, N-C*H*-Ph₂). Elementaranalyse für C₂₇H₂₅AuN₂O(% Soll/Ist): C (54,92/54,60), H (4,27/4,24), N (4,74/4,65).

### Beispiel 6: [2-(4-Methoxyphenyl)ethin-1-yl](tri(furan-2-yl)phosphan)-gold(I) (F)

Ausbeute: 39,0 mg (0,069 mmol, 36%), gelbliche Kristalle
¹H NMR (400 MHz, d₆-DMSO) δ 3,75 (s, 3H, CH₃), δ 6,74 (m, 3H, ArH), δ 6,88 (m, 2H, ArH), 7,27 (m, 5H, ArH), 8,20 (m breit, 3H, ArH).
Elementaranalyse für C₂₁H₁₇AuO₄P (% Soll/Ist): C (45,02/44,83), H (2,88/2,79), N(0,00/0,00)

### Zellkultur und Toxizitätsbestimmungen

Antiproliferative Effekte an MCF-7- und HT-29-Zelllinien wurden nach einem etabliertem Verfahren bestimmt (Scheffler, H.; You, Y.; Ott, I. Comparative studies on the cytotoxicity, cellular and nuclear uptake of a series of chloro gold(I) phosphine complexes. Polyhedron 2010, 29, 66-69).

### TrxR und GR Inhibitionsassays

TrxR- (Thioredoxin-Reduktase) und GR (Glutathion-Reduktase) Hemmungen wurden bestimmt mit Hilfe eines etablierten Assays, (Ott, I.; et. al., J. Med. Chem. 2009, 52, 763-770.und Smith, A. D.; et. Al., Int. J. Vitam. Nutr. Res. 2001, 71, 87-92.).

### Messung des mitochondrialen Sauerstoffverbrauchs

Die Messung des mitochondrialen Sauerstoffverbrauchs wurde nach einer Vorschrift durchgeführt, die in R. Rubbiani et al., J. Med. Chem., 2010, 53, 8608-8618, beschrieben ist.

### Bestimmung der antiangiogenen Eigenschaften

Die antiangiogenen Eigenschaften wurden nach einem Verfahren bestimmt, das in C.Bagowski et al. Dalton Trans., 2009, 10799-10805, beschrieben ist.

### Ergebnisse:

Die erfindungsgemäßen Verbindungen zeigen eine hervorragende selektive Hemmung der TrxR, insbesondere auch im Vergleich zur GR, wie in der Tabelle 1 gezeigt.

**Tabelle 1. Hemmung der Enzyme TrxR und Gr durch die Gold(I)-Komplexe, jeweils angegeben als EC₅₀ Wert mit Standardabweichung, ermittelt aus mehreren unabhängigen Experimenten.**

| | **TrxR EC₅₀ (µM)** | **GR EC₅₀ (µM)** | |
|---|---|---|---|
| Beispiel 1 | 0,0474 ^{±0,0067} | 19,1^{±6,0} | (Referenz) |
| Beispiel 2 | 0,0446 ^{±0,0335} | 10,3^{±4,4} | |
| Beispiel 3 | 0,3365 ^{±0,1131} | 31,1^{±7,0} | (Referenz) |
| Beispiel 4 | 0,4230 ^{±0,1130} | 14,9^{±11,9} | (Referenz) |
| Beispiel 6 | 0,92^{±0,24} | 6,3^{±2,2} | |

Die anti-proliferative Wirkung der Verbindungen ist in der Tabelle 2 dargestellt.

**Tabelle 2. Antiproliferatives Potential der Gold(I)-Komplexe. Angegeben sind die IC₅₀ Werte mit Standardabweichung, ermittelt aus mehreren unabhängigen Experimenten.**

| | **IC₅₀(HT-29) [µM]** | **IC₅₀(MCF-7) [µM]** | |
|---|---|---|---|
| Beispiel 1 | 5,2^{±1,5} | 0,8^{±0,5} | (Referenz) |
| Beispiel 2 | 50^{±0,3} | 1,0^{±0,2} | |
| Beispiel 3 | 12,0^{±3,5} | 2,2^{±0,6} | (Referenz) |
| Beispiel 4 | 4,5^{±0,3} | 0,8^{±0,3} | (Referenz) |
| Beispiel 5 | 9,0^{±3,1} | 8,6^{±2,8} | (Referenz) |
| Beispiel 6 | 3,8^{±0,4} | 4,5^{±0,3} | |

Die Angiogenese hemmenden Eigenschaften werden aus der Tab.3 deutlich. Unter Einwirkung von 0,1 bis 1,0 µM der beschriebenen Verbindungen wiesen die Zebrafischembryos nach 48 und 72 Stunden zu mehr als 90% sichbare Defekte in der Blutgefäßneubildung.

**Tabelle 3. Antiangiogene Eigenschaften der Goldkomplexe gemäß Beispiel 1 und Beispiel 2 an Zebrafisch-Embryos 48 Stunden und 72 Stunden nach der Befruchtung. Thalidomid ist als Referenz angegeben.**

| | **Konz. [µM]** | **Embryos mit Gefäßdefekten 48 h nach der Befruchtung [%]** | **Embryos mit Gefäßdefekten 72 h nach der Befruchtung [%]** |
|---|---|---|---|
| Beispiel 1 | 0,1 | 100^{±0} | 100^{±0} (Referenz) |
| Beispiel 1 | 1 | 100^{±0} | 100^{±0} (Referenz) |
| Beispiel 2 | 0,1 | 91^{±5} | 100^{±0} |
| Beispiel 2 | 1 | 98^{±2} | 91^{±5} |
| Thalidomid (rac.) | 0,1 | 56^{±9} | 56^{±9} |
| Thalidomid (rac.) | 1 | 55^{±7} | 55^{±7} |

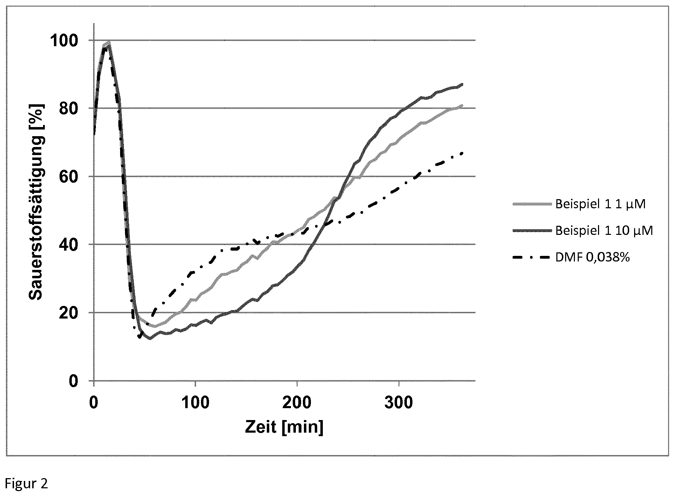

Die Effekte der Verbindung gemäß Beispiel 1 auf die mitochondriale Atmung ist in der Figur 2 dargestellt. DMF ist das Lösungsmittel alleine.

## Patentansprüche

1. Verbindungen der allgemeinen Formel (I): 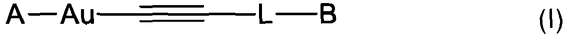
wobei A ein Substituent ist ausgewählt aus der Gruppe (II), (III) oder (IV): 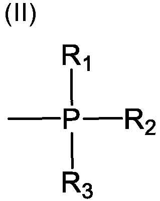 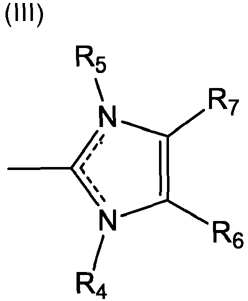 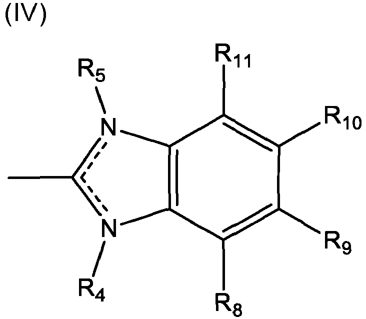
wobei R₁, R₂ und R₃ unabhängig voneinander ausgewählt ist aus CH₂-CH₃, einem gegebenenfalls substituiertem Phenylrest oder einem gegebenenfalls substituiertem Furanylrest, wobei dieser substituiert sein kann mit Substituenten ausgewählt aus Halogen, C₁-C₃ Alkyl, Hydroxyl, OC₁-C₃ Alkyl, -NH₂, -N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂,
wobei R₄, R₅, R₆, R₇, R₈, R₉, R₁₀, R₁₁, unabhängig voneinander ausgewählt ist aus der Gruppe bestehend aus Wasserstoff, Hydroxyl, linearen oder verzweigten C₁-C₆ Alkyl, Hydroxy-C₁-C₆ Alkyl, C₁-C₆ Alkoxy, C₁-C₆ Alkyl-C₁-C₆ Alkoxy, Benzyl, oder gegebenenfalls substituierten aromatischen Rest, wobei dieser aromatische Rest substituiert sein kann mit Substituenten ausgewählt aus Halogen, C₁-C₃ Alkyl, Hydroxyl, OC₁-C₃ Alkyl, -NH₂,-N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂,
L stellt eine Linkergruppe dar, die fehlt.
B ist ein gegebenenfalls substituierter aromatischer Rest, wobei dieser aromatische Rest substituiert sein kann mit Substituenten ausgewählt aus C₁-C₃ Alkyl, Hydroxyl, OC₁-C₃ Alkyl, -NH₂, -N(C₁-C₃ Alkyl)₂, -NO₂, SC₁-C₃ Alkyl, C₁-C₃ Alkoxy, C₁-C₆ Alkyl-N(C₁-C₆ Alkyl)₂, C₁-C₆ Alkyl-NH-C₁-C₆ Alkyl, C₁-C₆ AlkylNH₂, Halogen,
und deren pharmazeutisch annehmbare Salze zur Verwendung als Angiogenesehemmer zur Behandlung von Krebs.

2. Verbindungen zur Verwendung als Angiogenesehemmer zur Behandlung von Krebs nach Anspruch 1, wobei B ein Aromat ist ausgewählt aus Benzol, Anthracen, Phenanthren, der gegebenenfalls substituiert sein kann.

3. Verbindungen zur Verwendung als Angiogenesehemmer zur Behandlung von Krebs gemäß der allgemeinen Formel (I) nach einem der vorherigen Ansprüche, **dadurch gekennzeichnet, dass** A ein Substituent der allgemeinen Formel (III) ist mit R₄ und R₅ ausgewählt aus Methyl, Ethyl, Propyl oder Butyl.

4. Verbindung zur Verwendung als Angiogenesehemmer zur Behandlung von Krebs nach Anspruch 1, **dadurch gekennzeichnet, dass** diese eine der Verbindungen [2-(4-Methoxyphenyl)ethin-1-yl](triphenylphosphan)-gold(I) (B), oder [2-(4-Methoxyphenyl)ethin-1-yl](tri(furan-2-yl)phosphan)-gold(I) (F) ist.

5. Pharmazeutische Zusammensetzung enthaltend eine Verbindung der allgemeinen Formel (I), wie in Anspruch 1 definiert, wobei A ein Substituent der allgemeinen Formel (III) oder (IV) oder eine Verbindung der allgemeinen Formel (I), wobei A ein Substituent der allgemeinen Formel (II), wobei R₁, R₂ und R₃ ein gegebenenfalls substituierter Furanylrest ist.

## Claims

1. Compounds of the general formula (I): 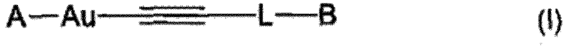
where A is a substituent selected from the group (II), (III) or (IV): 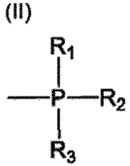 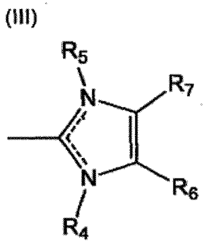 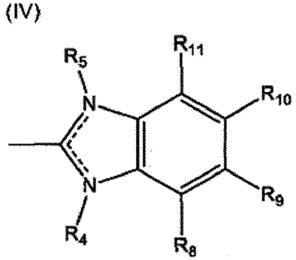
where R₁, R₂ and R₃ are each independently selected from CH₂-CH₃, an optionally substituted phenyl radical or an optionally substituted furanyl radical, in which these may be substituted by substituents selected from halogen, C₁-C₃ alkyl, hydroxyl, OC₁-C₃ alkyl, -NH₂, -N(C₁-C₃ alkyl)₂, -NO₂, SC₁-C₃ alkyl, C₁-C₃ alkoxy, C₁-C₆ alkyl-N(C₁-C₆ alkyl)₂, C₁-C₆ alkyl-NH-C₁-C₆ alkyl, C₁-C₆ alkylNH₂, where R₄, R₅, R₆, R₇, R₈, R₉, R₁₀, R₁₁ are each independently selected from the group consisting of hydrogen, hydroxyl, linear or branched C₁-C₆ alkyl, hydroxyl-C₁-C₆ alkyl, C₁-C₆ alkoxy, C₁-C₆ alkyl-C₁-C₆ alkoxy, benzyl, or optionally substituted aromatic radical, wherein this aromatic radical may be substituted by substituents selected from halogen, C₁-C₃ alkyl, hydroxyl, OC₁-C₃ alkyl, -NH₂, -N(C₁-C₃ alkyl)₂, -NO₂, SC₁-C₃ alkyl, C₁-C₃ alkoxy, C₁-C₆ alkyl-N(C₁-C₆ alkyl)₂, C₁-C₆ alkyl-NH-C₁-C₆ alkyl, C₁-C₆ alkylNH₂,
L is a linking group, which is not present,
B is an optionally substituted aromatic radical, wherein this aromatic radical may be substituted by substituents selected from C₁-C₃ alkyl, hydroxyl, OC₁-C₃ alkyl, -NH₂, -N(C₁-C₃ alkyl)₂, -NO₂, SC₁-C₃ alkyl, C₁-C₃ alkoxy, C₁-C₆ alkyl-N(C₁-C₆ alkyl)₂, C₁-C₆ alkyl-NH-C₁-C₆ alkyl, C₁-C₆ alkylNH₂, halogen, and pharmaceutically acceptable salts thereof for use as angiogenesis inhibitors for the treatment of cancer.

2. Compounds for use as angiogenesis inhibitors for the treatment of cancer according to Claim 1, wherein B is an aromatic system selected from benzene, anthracene, phenanthrene, which may optionally be substituted.

3. Compounds for use as angiogenesis inhibitors for the treatment of cancer according to the general formula (I) according to either of the preceding claims, **characterized in that** A is a substituent of the general formula (III) where R₄ and R₅ are selected from methyl, ethyl, propyl or butyl.

4. Compound for use as angiogenesis inhibitor for the treatment of cancer according to Claim 1, **characterized in that** said compound is one of the compounds [2-(4-methoxyphenyl)ethyn-1-yl](triphenylphosphane)gold(I) (B), or [2-(4-methoxyphenyl)ethyn-1-yl](tri(furan-2-yl)phosphane)gold(I) (F).

5. Pharmaceutical composition comprising a compound of the general formula (I), as defined in Claim 1, wherein A is a substituent of the general formula (III) or (IV), or a compound of the general formula (I), wherein A is a substituent of the general formula (II), where R₁, R₂ and R₃ are optionally substituted furanyl radicals.

## Revendications

1. Composés de formule générale (I) : 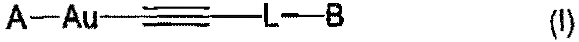
dans laquelle A est un substituant choisi dans le groupe constitué par (II), (III) ou (IV) : 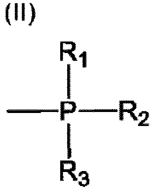 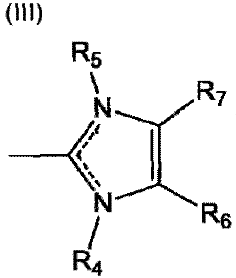 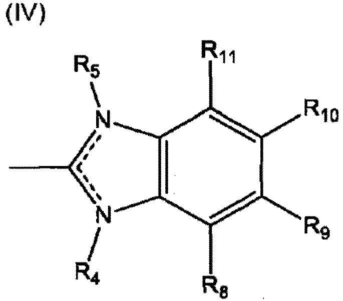
dans lesquelles R₁, R₂ et R₃ sont choisis indépendamment les uns des autres parmi CH₂-CH₃, un radical phényle éventuellement substitué ou un radical furanyle éventuellement substitué, celui-ci pouvant être substitué avec des substituants choisis parmi halogène, alkyle en C₁-C₃, hydroxyle, O-alkyle en C₁-C₃, -NH₂,-N(alkyle en C₁-C₃)₂, -NO₂, S-alkyle en C₁-C₃, alcoxy en C₁-C₃, alkyle en C₁-C₆-N(alkyle en C₁-C₆)₂, alkyle en Ci-C₆-NH-alkyle en C₁-C₆, alkyle en C₁-C₆-NH₂,
R₄, R₅, R₆, R₇, R₈, R₉, R₁₀, R₁₁ sont choisis indépendamment les uns des autres dans le groupe constitué par hydrogène, hydroxyle, alkyle en C₁-C₆ linéaire ou ramifié, hydroxy-alkyle en C₁-C₆, alcoxy en C₁-C₆, alkyle en C₁-C₆-alcoxy en C₁-C₆, benzyle ou un radical aromatique éventuellement substitué, ce radical aromatique pouvant être substitué avec des substituants choisis parmi halogène, alkyle en C₁-C₃, hydroxyle, O-alkyle en C₁-C₃, -NH₂, -N(alkyle en C₁-C₃)₂, -NO₂, S-alkyle en C₁-C₃, alcoxy en C₁-C₃, alkyle en C₁-C₆-N(alkyle en C₁-C₆)₂, alkyle en C₁-C₆-NH-alkyle en C₁-C₆, alkyle en C₁-C₆-NH₂,
L représente un groupe de liaison qui est absent,
B est un radical aromatique éventuellement substitué, ce radical aromatique pouvant être substitué avec des substituants choisis parmi alkyle en C₁-C₃, hydroxyle, 0-alkyle en C₁-C₃, -NH₂, -N(alkyle en C₁-C₃)₂, -NO₂, S-alkyle en C₁-C₃, alcoxy en C₁-C₃, alkyle en C₁-C₆-N(alkyle en C₁-C₆)₂, alkyle en C₁-C₆-NH-alkyle en C₁-C₆, alkyle en C₁-C₆-NH₂, halogène
et leurs sels pharmaceutiquement acceptables, destinés à une utilisation en tant qu'inhibiteur de l'angiogenèse pour le traitement du cancer.

2. Composés destinés à une utilisation en tant qu'inhibiteur de l'angiogenèse pour le traitement du cancer selon la revendication 1, dans lesquels B est un composé aromatique choisi parmi le benzène, l'anthracène, le phénanthrène, qui peut éventuellement être substitué.

3. Composés destinés à une utilisation en tant qu'inhibiteur de l'angiogenèse pour le traitement du cancer selon la formule générale (I) selon l'une quelconque des revendications précédentes, **caractérisés en ce qu'**A est un substituant de formule générale (III) avec R₄ et R₅ choisis parmi méthyle, éthyle, propyle ou butyle.

4. Composé destiné à une utilisation en tant qu'inhibiteur de l'angiogenèse pour le traitement du cancer selon la revendication 1, **caractérisé en ce que** celui-ci est un des composés [2-(4-méthoxyphényl)éthin-1-yl](triphénylphosphane)-or (I) (B) ou [2-(4-méthoxyphényl)éthin-1-yl](tri(furan-2-yl)phosphane)-or (I) (F).

5. Composition pharmaceutique contenant un composé de formule générale (I), telle que définie dans la revendication 1, dans laquelle A est un substituant de formule générale (III) ou (IV) ou un composé de formule générale (I) dans laquelle A est un substituant de formule générale (II), R₁, R₂ et R₃ étant un radical furanyle éventuellement substitué.
